# EUROPEAN PATENT APPLICATION

(11) **EP 3 696 281 A1**
(43) Date of publication of application: **19.08.2020**
(21) Application number: 19156908.6
(22) Date of filing: 13.02.2019
(51) Int. Cl.: C12Q 1/6883, G01N 33/50

(54) **ARGINASE AS BIOMARKER AND DRUG TARGET FOR PCOS**

(71) Applicant: Johann Wolfgang Goethe-Universität Frankfurt am Main, 60323 Frankfurt am Main (DE)
(72) Inventor: Randriamboavonjy, Voahanginirina, 60529 Frankfurt am Main (DE)
(74) Representative: Kuttenkeuler, David

(57) **Abstract**

The invention is based on the surprising finding that in women with polycystic ovary syndrome (PCOS) an increased activity and/or expression of arginase is detected. The invention uses therefore various arginase associated biomarkers as indicators for the occurrence with the disease. More importantly the invention describes genetic variations associated with increased arginase expression/activity, and further being associated with PCOS. Hence, the invention provides enzymatic, immunological, and genetic diagnostic approaches for the detection and monitoring of PCOS in a subject. Furthermore the invention shows that the causal link of PCOS and arginase activity and hence provides the use of arginase inhibitors as a treatment approach.

## Description

### FIELD OF THE INVENTION

The invention is based on the surprising finding that in women suffering from polycystic ovary syndrome (PCOS) an increased activity and/or expression of arginase is detected. The invention uses therefore various arginase associated biomarkers as indicators for the occurrence with the disease. More importantly the invention describes genetic variations associated with increased arginase expression/activity, and further being associated with PCOS. Hence, the invention provides enzymatic, immunological, and genetic diagnostic approaches for the detection and monitoring of PCOS in a subject. Furthermore the invention shows the causal link of PCOS and arginase activity and hence provides the use of arginase inhibitors as a treatment approach.

### DESCRIPTION

Polycystic ovary syndrome (PCOS) is one of the most common endocrinopathies in women of reproductive-age with a worldwide prevalence of 10-15% ^{[1-2]}. The etiology and pathophysiology of PCOS is unclear but it is considered to be a multifactorial disease involving genetic, endocrine and metabolic factors ^{[3]}. Clinically, hyperandrogenism, menstrual dysfunction and polycystic-appearing ovaries characterize the syndrome. PCOS is also associated with insulin resistance and an increased risk of developing type 2 diabetes as well as cardiovascular diseases ^{[4]}.

Today, there is no drug specifically approved for the indication of PCOS and therapies are mostly focused to treat the accompanying symptoms and to improve fertility. Ovulation-inducers such as clomiphene citrate or letrozole are the first-line medications for anovulatory infertility ^{[5]}. However, given the number of patients who are resistant to these therapies and the multifactorial causes of PCOS-associated infertility, other approaches have been introduced ^{[6]}. For example, insulin-sensitizing drug such as metformin has been used to treat PCOS patients and has been reported to improve fertility ^{[7]}. However, metformin is not well-tolerated by a number of patients and is associated with different adverse effects. Given the complexity of the syndrome and the lack of real therapy, studies are necessary to better understand the pathogenesis of PCOS in order to allow the development of a targeted therapy that is effective not only in improving fertility but also in reducing PCOS-associated cardiovascular complications.

The aim of the present invention was therefore to decipher the pathophysiology of PCOS and to develop both novel diagnostic and treatment options for PCOS.

### BRIEF DESCRIPTION OF THE INVENTION

Generally, and by way of brief description, the main aspects of the present invention can be described as follows:
In **a first aspect,** the invention pertains to a method for diagnosing and/or monitoring polycystic ovary syndrome (PCOS) in a subject, the method comprising the steps of:
   **(a)** Providing one or more biological samples of the subject to be diagnosed,
   **(b)** Determining in the one or more biological samples one or more of the following biomarkers:
      (i) The level of ornithine, or a metabolic derivative of ornithine, wherein the metabolic derivative of ornithine is indicative for the level of ornithine,
      (ii) The level of arginine, or a metabolic derivative of arginine, wherein the metabolic derivative of arginine is indicative for the level of arginine,
      (iii) The ratio of the levels as determined in (i) and (ii),
      (iv) The level of, or activity of, arginase,
      (v) The level of arginase-bearing microparticles,
      (vi) The presence or absence of a Single Nucleotide Polymorphism (SNP) in an arginase gene;
   **(c)** Comparing the one or more biomarker determined in (b) with a predetermined references value of said one or more biomarker which is indicative for the absence of PCOS; and/or comparing the one or more biomarker determined in (b) with a reference value of said biomarker determined in a biological sample of a healthy subject;
   wherein if the one or more biomarker as determined in (b) significantly deviates from the reference or control value, the subject is diagnosed with PCOS, and/or the monitored subject is determined to suffer from PCOS and to still require treatment.
In **a second aspect,** the invention pertains to a diagnostic kit for the detection of PCOS in a subject, wherein the kit comprises means for the detection of one or more biomarkers selected from, the level of arginine or ornithine, or metabolic derivatives thereof indicative for the level of arginine and/or ornithine, the activity or level of arginase, the level of arginase-bearing microparticles, the presence of an SNP selected from rs2781666 and rs2781667, preferably of both, or combinations thereof, and optionally, instructions for use of the kit.
In **a third aspect,** the invention pertains to an arginase inhibitor for use in the treatment of PCOS.
In **a fourth aspect,** the invention pertains to a pharmaceutical composition for use in the treatment of PCOS, comprising an arginase inhibitor and a pharmaceutically acceptable carrier and/or excipient.
In **a fifth aspect,** the invention pertains to a use of an arginase inhibitor for the manufacturing of a medicament for the treatment of PCOS.
In **a sixth aspect,** the invention pertains to a method of treatment of PCOS in a subject suffering from or suspected to suffer from PCOS, wherein the method comprises the step of administering to the subject a therapeutically effective amount of an arginase inhibitor.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the invention pertains to a method for diagnosing polycystic ovary syndrome (PCOS) in a subject, the method comprising the steps of:
(a) Providing one or more biological samples of the subject to be diagnosed,
(b) Determining in the one or more biological samples one or more of the following biomarkers:
   (i) The level of ornithine, or a metabolic derivative of ornithine, wherein the metabolic derivative of ornithine is indicative for the level of ornithine,
   (ii) The level of arginine, or a metabolic derivative of arginine, wherein the metabolic derivative of arginine is indicative for the level of arginine,
   (iii) The ratio of the levels as determined in (i) and (ii),
   (iv) The level of, or activity of, arginase,
   (v) The level of arginase-bearing microparticles,
   (vi) The presence or absence of a Single Nucleotide Polymorphism (SNP) in an arginase gene;
(c) Comparing the one or more biomarker determined in (b) with a predetermined references value of said one or more biomarker which is indicative for the absence of PCOS; and/or comparing the one or more biomarker determined in (b) with a reference value of said biomarker determined in a biological sample of a healthy subject;
wherein if the one or more biomarker as determined in (b) significantly deviates from the reference or control value, the subject is diagnosed with PCOS.

In a preferred embodiment, the term "diagnosis" means both the detection of the disease in a subject suspected of having PCOS and the monitoring of the progress of the disease and/or the monitoring of the effectiveness of the therapy in a subject already diagnosed as having PCOS. Therefore, the term "subject" means, as the case may be, a subject suspected of having PCOS or a subject already diagnosed as having PCOS.

The person skilled in the art will appreciate that a clinician does usually not conclude whether or not the patient suffers or is likely to suffer from a disease, condition or disorders, such as PCOS, solely on the basis of a single diagnostic parameter, but needs to take into account other aspects, for example the presence of autoantibodies, markers, blood parameters, clinical assessment of the patient's symptoms or the results of medical imaging or other non-invasive methods such as polysomnography, to arrive at a conclusive diagnosis. The value of a diagnostic agent or method may also reside the possibility to rule out one disease, thus allowing for the indirect diagnosis of another.

Therefore, the term "diagnosis" does preferably not imply that the diagnostic methods according to the present invention will be definitive and sufficient to finalize the diagnosis of PCOS on the basis of a single test, let alone parameter, but may refer to a contribution to what is referred to as a "differential diagnosis" of PCOS, i. e. a systematic diagnostic procedure, considering the likelihood of a range of possible conditions on the basis of a range of diagnostic parameters available to the skilled artisan at the time. The term "diagnosis" may also refer to a method used to distinguish between two or more conditions associated with similar or identical symptoms. The term "diagnosis" may also refer to a method used to choose the most promising treatment regime for a patient. In other words, the method may relate to selecting a treatment regimen for a subject. Such treatment may comprise the administration of drugs known to slow down the progression of PCOS, and are preferably selected from the herein disclosed arginase inhibitors.

A subject in context of the herein disclosed invention is preferably a mammal, more preferably a female mammal, such as a female human (woman) suspected to suffer from PCOS, or showing to some degree symptoms of PCOS.

In preferred embodiments, the one or more biological sample is selected from a tissue sample such as vaginal smear, oocytes, or a liquid sample, such as a blood sample (whole blood preferably), serum sample or plasma sample. In particular embodiments, the method comprising a step of determining of the biomarkers (i) to (v), preferably comprises the use of a blood sample (whole blood preferably), or blood-derived sample, and preferably a plasma sample is provided. In other embodiments, the method when pertaining to determining the biomarker (vi) preferably comprises the use of a DNA containing sample as biological sample of the subject, preferably such biological sample comprises genetic material, such as cells, and preferably is a sample comprising platelets, preferably wherein the sample is a whole blood sample or buffy coat sample containing leukocytes.

The term "determining" in context of the invention shall have a meaning corresponding to "detect" or "measure" a level or a presence or absence of a biomarker according to (i) to (vi) of the method of the invention. Hence, "determining" shall pertain to identifying the presence, absence, amount, or level of the object to be detected (for example, a biomarker according to the invention). As used herein, the term "level" refers to the amount of or concentration of a biomarker. Typically, the level of the marker in a biological sample obtained from the subject is different (for example, increased or decreased) from a predetermined level (for example, the level of the same variant in a similar sample obtained from a healthy individual. Such a predetermined level is referred to as a "reference value" or "reference level" or similar terms.

As used herein, "predetermined level" refers to the level of expression of or concentration or activity of a biomarker or to a ratio of biomarkers in a control sample (for example, a biological sample from a subject without PCOS). In some embodiments, PCOS can be diagnosed by assessing whether the biomarker level or ratio of biomarkers varies from a predetermined level. For instance, the difference may be greater than, less than, equal to, or any number in between about 5%, 10%, 15%, 20%, 25%, 30%, 35%, 40%, 45%, 50%, 55%, 60%, 65%, 70%, 75%, 80%, 85%, 90%, 95%, 100%, 110%, 125%, 150%, 175%, 200%, 250%, 300%, 350%, 400%, 450%, 500%, 550%, 600%, 650%, 700%, 750%, 800%, 850%, 900%, 950%, 1,000%, 5,000%, 10,000%, 100,000% or greater. The predetermined level can be determined from a control. A control can be a sample or its equivalent from a normal patient or from a patient in a known disease state. For instance, the control can be from a woman not having PCOS, or an average of multiple women not having PCOS. The control can also be a standard or known amount of a reference biomarker (for example, a biomarker according to (i) to (vi)) or a standard or known amount of a ratio of biomarkers.

In some embodiments, the herein disclosed methods and uses are not performed directly at the patient's body, but are preferably *in vitro* or *ex vivo* methods.

One of the biomarker of the invention is ornithine (preferably L-ornithine). Ornithine has the following structure: 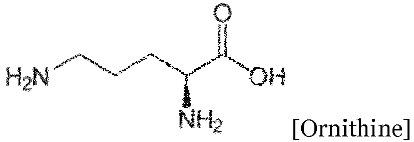 and is a non-proteinogenic amino acid, having biological functions in the urea cycle. L-Ornithine is one of the products of the action of the enzyme arginase on L-arginine, creating urea. Therefore, ornithine is a central part of the urea cycle, which allows for the disposal of excess nitrogen. In context of the herein disclosed invention, for a diagnosis of PCOS, the level of ornithine, or a metabolic derivative of ornithine, is determined, wherein the metabolic derivative of ornithine is indicative for the level of ornithine Hence, in preferred embodiments the term "metabolic derivative of ornithine" shall pertain to such compounds which are metabolic precursors or metabolites of, or degradation products of, ornithine that have the ability when the level of such metabolic derivative of ornithine is determined in a sample of a subject, it allows to deduce therefrom the level of ornithine in the subject. Hence, the metabolic derivative of ornithine preferably allows for the indirect demining of the level of ornithine in the subject.

In preferred of some particular embodiments, for the biomarker in (i) an increase of the level of ornithine, or the metabolic derivative of ornithine, compared to the reference value indicates the presence of PCOS in the subject.

One of the biomarker of the invention is arginine (preferably L-arginine). Arginine has the following structure: 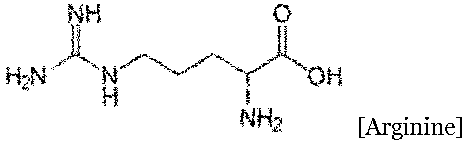 and is a proteinogenic amino acid, having biological functions in the urea cycle. L-Arginine is one of the educts of the action of the enzyme arginase for the production of L-ornithine and urea. Therefore, ornithine is a central part of the urea cycle, which allows for the disposal of excess nitrogen. In context of the herein disclosed invention, for a diagnosis of PCOS, the level of arginine, or a metabolic derivative of arginine, is determined, wherein the metabolic derivative of arginine is indicative for the level of arginine. Hence, in preferred embodiments the term "metabolic derivative of arginine" shall pertain to such compounds which are metabolic precursors or metabolites of, or degradation products of, arginine that have the ability when the level of such metabolic derivative of arginine is determined in a sample of a subject, it allows to deduce therefrom the level of arginine in the subject. Hence, the metabolic derivative of arginine preferably allows for the indirect demining of the level of arginine in the subject. Ornithine is the most prominent example of an arginine metabolite.

In preferred of some particular embodiments, for the biomarker in (ii) a decrease of the level of arginine or the metabolic derivative of arginine, compared to the reference value indicates the presence of PCOS in the subject.

As an additional biomarker, according to the PCOS diagnosis of the invention, in (iii) an increase of the ratio of [the value determined in (i)]: [the value determined in (ii)] indicates the presence of PCOS. Therefore, the ratio of the level or ornithine to arginine in the sample of the subject is indicative for the presence or absence of PCOS of the subject. Without being bound to theory, the increase of the ratio as defined herein is thought to indicate an increase in arginase activity (arginase protein expression or arginase enzymatic activity).

The detection of ornithine or arginine, or their ratio, as well as any metabolic derivative of these molecules, does in particular embodiments involve mass spectrometry, or colorimetric/fluorometric assays. Such assays for the quantification of amino acids are well-known to the skilled biochemist.

The term "arginase" in context of the invention shall refer in preferred embodiments to an arginase protein, an arginase encoding gene, or an arginase mRNA, depending on the context. Arginase proteins generally are polypeptides that catalyze the hydrolysis of arginine to form urea and ornithine. An arginase protein according to the invention in preferred embodiments is human arginase 1 (ARGi), preferably having an amino acid sequence according to SEQ ID NO:1, or an isoform thereof, or is human arginase 2 (ARG2), preferably having an amino acid sequence according to SEQ ID NO:2, or an isoform thereof. Information on the gene and protein of arginase 1 and 2 can be derived for example from the Human Gene Nomenclature Committee (HGNC) database (www.genenames.org; in the version of February 12, 2019). ARG1 has the ID HGNC:663, whereas ARG2 has the ID HGNC:664. Information about the protein and its isoforms may be derived from the UniProt database (www.uniprot.org; UniProt: the universal protein knowledgebase, Nucleic Acids Res. 46: 2699 (2018)). ARG1 has the UniProt ID of: P05089, whereas ARG2 has the UniProt ID of: P78540.

In context of the herein disclosed invention for the biomarker in (iv) an increase of the level of, or activity of, arginase, compared to the reference value indicates the presence of PCOS.

The level of an arginase in context of the invention may be a level of arginase protein or arginase mRNA in a sample. Determining such level might involve any methods known for protein or RNA detection and quantification including but not limited to PCR based techniques, immunological methods, such as ELISA, or mass spectrometry based approaches and many more. For immunological assays the use of arginase specific antibodies, or antibody-based binding proteins, is preferred.

In addition, also the arginase activity in a sample may be detected, for example using enzymatic assays. The term "arginase activity" refers to an enzymatic activity of an arginase, or a mutein, homologous protein, analog, derivative, fusion or fragment thereof, that catalyzes the conversion of arginine (arg) to ornithine. Arginase enzymatic assays are well known to those of skill in the art. For example, the arginase activity assay is selected from the Arginase Activity Colorimetric Assay Kit (BioVision™ Arginase assay; or Arginase Activity Assay Kit (Colorimetric) from abcam™ (ab180877)). Alternatively or additionally, the activity of arginase can be assayed with other biochemical assays, such as detection of urea (MAK112®). In context of the invention, an increase in enzymatic activity in the sample compared to the reference values indicates the PCOS in the subject.

Arginase may, in context of the invention, also be detected by way of detecting arginase bearing micro-particles. Detection of particles bearing arginase, such as exosomes, is in principle done in a similar fashion as described herein for the detection or determining of arginase level or activity. Preferably, an increase of the level of arginase-bearing microparticles compared to the reference value indicates the presence of PCOS in the subject.

In particular embodiments, the method of diagnosing PCOS according to the invention involves detection of polymorphisms in the genes for arginase of the subject to be diagnosed. The term "genetic polymorphism" refers to a variation in nucleotide sequence of the strand of DNA having at least one frequency of 1% in a population of individuals. Genetic polymorphisms may be variations of one or more nucleotides. The single nucleotide polymorphism, "single nucleotide polymorphism" or SNP, generally gives rise to up to three, usually two, different alleles. In the present invention the term "polymorphism" and "SNP" are used interchangeably. In context of the invention, the genetic polymorphism is associated with an increased expression or activity of an arginase. Preferably but not necessarily, the SNP of the invention is located within the gene of arginase, preferably human arginase 1. In this context, the SNP is a genetic polymorphism in the arginase gene and which increases the expression or activity of arginase in the individual having the SNP compared to an individual not having the SNP.

In context of the invention, the SNP is preferably selected from rs2781666 and rs2781667, preferably of the thymine (t) allele of said SNPs, and more preferably, wherein the presence or absence of both SNPs is determined for a diagnosis of PCOS. Hence, for the biomarker in (vi) the presence of one of the t alleles of the SNPs, preferably of both, indicates the presence of PCOS. The presence or absence of the SNP in the sample of the subject is preferably detected by nucleic acid hybridization (SNP arrays), gene sequencing (next generation sequencing) or PCR based assays. Any method useful for the detection of SNPs can be used in the present invention, and many different methods are known in the art for SNP genotyping (for review see Syvanen, A. C. (2001); Kim, S. and Misra, A., (2007)). Such methodology may consist of the use of three steps in succession, including a "reaction" (e.g., hybridization, ligation, extension and cleavage) followed by "separation" (e.g., solid phase microtitre plates, microparticles or arrays, gel electrophoresis, solution-phase homogenous or semi -homogenous). No single ideal SNP genotyping method exists for all applications, and it is well within the skill of a skilled artisan to determine the most appropriate method given the various parameters, such as sample size and number of SNPs to be analysed.

In a second aspect the invention provides a diagnostic kit for the detection of PCOS in a subject, wherein the kit comprises means for the detection of one or more biomarkers selected from, the level of arginine or ornithine, or metabolic derivatives thereof indicative for the level of arginine and/or ornithine, the activity or level of arginase, the level of arginase-bearing microparticles, the presence of an SNP selected from rs2781666 and rs2781667, preferably of both, or combinations thereof, and optionally, instructions for use of the kit. Preferably the kit of the invention is (suitable) for use in a method according to the herein described first aspect.

In a third aspect, the invention then provides an arginase inhibitor for use in the treatment of PCOS. Alternatively, the third aspect relates to a method of treating PCOS in a subject, comprising a step of administering an effective amount of an arginase inhibitor to the subject. Further alternatively, the third aspect of the invention relates to the use of an arginase inhibitor for the manufacture of a medicament for the treatment of PCOS in a subject.

The term "arginase inhibitor" shall refer in context of the invention to any compound that when administered to a patient affects a reduction of the activity of arginase in the patient. Hence such an arginase inhibitor may be selected from an antibody, or a derivative thereof, a small molecule, a nucleic acid based compound, such as a miRNA, siRNA, gene editing construct(s), or is an amino acid derivative, preferably an arginine or ornithine derivative.

The arginase inhibitor of the invention in some embodiments is an arginase inhibitor that when contacted with an arginase enzyme has the ability to reduce the enzymatic activity. Alternatively, the arginase inhibitor when introduced into a cell reduces the expression of the arginase, for example by reducing activity of the arginase gene, or by interfering with translation (RNA interference).

Generally, for all aspects and embodiments of the herein disclosed invention, the activity of arginase shall be understood as an enzymatic activity catalysing the reaction: arginine + H2O → ornithine + urea.

Many arginase inhibitors are known to the skilled artisan and are useful also in context of the herein disclosed medical treatments. Such inhibitors include 2(S)-amino-6-boronohexanoic acid (ABH), Nω-OH-L-arginine (NOHA), Nω-hydroxy-nor-L-arginine (nor-NOHA), α-difluoromethylornithine (DFMO), L-norvaline, iodoacetyl-L-ornithine, iodoacetyl-L-lysine, L-lysine, and L-citrulline, (2s)-(+)-amino-5-iodoacetamidopentanoic acid, NG-hydroxy-L-arginine, (2S)-(+)-amino-6-iodoacetamidohexanoic acid, and (R)-2-amino-6-borono-2-(2-(piperidin-1-yl)ethyl)hexanoic acid, the small arginase inhibitor INCB001158 from Calithera or a new small molecule inhibitor of arginase or a combination thereof, and any pharmaceutical acceptable salts or solvates, thereof.

The medical uses and treatments of the invention preferably comprise that the arginase inhibitor is administered in a therapeutically effective amount to a subject suffering from, or suspected to suffer from, PCOS. As used herein, the term "administering" refers to oral, topical, parenteral, intravenous, intraperitoneal, intramuscular, intralesional, intranasal, subcutaneous, or intrathecal administration to a subject, as well administration as a suppository or the implantation of a slow-release device, e.g., a mini-osmotic pump, in the subject.

As used herein, the terms "treatment" and "treating" refer to full or partial treatment or amelioration of the pathology, condition, or symptom of e.g., PCOS, including any objective or subjective parameter such as abatement; remission; diminishing of symptoms or making the symptom, pathology or condition more tolerable to the patient; decreasing the frequency or duration of the symptom or condition; or, in some situations, preventing the onset of symptoms. The treatment or amelioration of symptoms can be based on any objective or subjective parameter; including, e.g., the result of a physical examination.

As used herein, the terms "therapeutically effective amount" refers to a dose of a drug or other agent that produces therapeutic effects for which it is administered. The exact dose will depend on the purpose of the treatment, and will be ascertainable by one skilled in the art using known techniques (see, e.g., Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins). In sensitized cells, the therapeutically effective dose can often be lower than the conventional therapeutically effective dose for non-sensitized cells.

Typically dosages of the arginase inhibitor which may be administered to a subject suffering from PCOS, preferably a human, range in amount from 1 µg to about 100 g per kilogram of body weight of the subject. While the precise dosage administered will vary depending upon any number of factors, including but not limited to, the type of animal and type of PCOS state being treated, the age of the subject and the route of administration. Preferably, the dosage of the compound will vary from about 1 mg to about 10 g per kilogram of body weight of the subject. More preferably, the dosage will vary from about 10 mg to about 1 g per kilogram of body weight of the animal.

The compound may be administered to an animal as frequently as several times daily, or it may be administered less frequently, such as once a day, once a week, once every two weeks, once a month, or even less frequently, such as once every several months or even once a year or less. The frequency of the dose will be readily apparent to the skilled artisan and will depend upon any number of factors, such as, but not limited to, the type and severity of the disease being treated, the type and age of the animal, etc.

The treatment of the invention in some embodiments that are preferred involves additionally obtaining a biological sample of a subject suspected to suffer from PCOS, diagnosing PCOS in the subject according to the method of the first aspect described herein using said biological sample, and if the subject is diagnosed with PCOS, administering a therapeutically effective amount of the arginase inhibitor to the subject.

In other embodiments of the invention the herein disclosed treatment may preferably involve the steps of monitoring the level or activity of arginase in a biological sample obtained from the subject to be treated, and adjusting the amount of administered arginase inhibitor to reduce the level or activity of arginase in the subject to a therapeutically effective level. Preferably a therapeutically effective level of arginase level or activity is the level of or activity of arginase in a healthy subject.

In **a fourth aspect,** the invention pertains to a composition comprising an arginase inhibitor, together with a carrier and/or excipient, for use in the treatment of PCOS.

The composition which is suitable for use in medicine is preferably a pharmaceutical composition, and a composition wherein the carrier and/or excipient are pharmaceutically acceptable, preferably in a form suitable for administration of the composition to a subject.

The present invention further includes various pharmaceutical compositions comprising arginase inhibitors contemplated herein and a pharmaceutically acceptable carrier and/or excipient. Most preferably the pharmaceutical compositions of the invention comprise an arginase inhibitor of the invention as described before. As used herein "pharmaceutically acceptable carrier" includes any and all solvents, dispersion media, coatings, antibacterial and antifungal agents, isotonic and absorption delaying agents, and the like that are physiologically compatible, including pharmaceutically acceptable cell culture media. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the vectors contemplated herein, use thereof in the pharmaceutical compositions of the invention is also contemplated.

The compositions of the invention may comprise one or more polypeptides, polynucleotides, and vectors comprising same, transduced cells, etc., as described herein, formulated in pharmaceutically-acceptable or physiologically-acceptable solutions for administration to a cell or an animal, either alone, or in combination with one or more other modalities of therapy. It will also be understood that, if desired, the compositions of the invention may be administered in combination with other agents as well, such as, e.g., cytokines, e.g., anti-inflammatory cytokines, growth factors, hormones, small molecules or various pharmaceutically-active agents. There is virtually no limit to other components that may also be included in the compositions, provided that the additional agents do not adversely affect the ability of the composition to deliver the intended (gene) therapy.

In the pharmaceutical compositions contemplated herein, formulation of pharmaceutically-acceptable excipients and carrier solutions is well-known to those of skill in the art, as is the development of suitable dosing and treatment regimens for using the particular compositions described herein in a variety of treatment regimens, including e.g., oral, parenteral, intravenous, intranasal, intramuscular, intrathecal, intraneural, intraganglion, intracranial, and intraventricular administration and formulation.

In certain circumstances it will be desirable to deliver the compositions disclosed herein parenterally, intravenously, intramuscularly, intraperitoneally, intrathecally, intraneurally, intraganglionicly, intracranially, or intraventricularly. Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms.

The compositions disclosed herein may be formulated in a neutral or salt form. Pharmaceutically-acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective. The formulations are easily administered in a variety of dosage forms such as injectable solutions, drug-release capsules, and the like.

As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The phrase "pharmaceutically-acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared. The preparation can also be emulsified.

In particular, the compositions of the present invention may be formulated for delivery either encapsulated in a lipid particle, a liposome, an exosome, a vesicle, a nanosphere, a nanoparticle or the like. The formulation and use of such delivery vehicles can be carried out using known and conventional techniques. The formulations and compositions of the invention may comprise one or more polypeptides, polynucleotides, and small molecules, as described herein, formulated in pharmaceutically-acceptable or physiologically-acceptable solutions (e.g., culture medium) for administration to a cell or an animal, either alone, or in combination with one or more other modalities of therapy. It will also be understood that, if desired, the compositions of the invention may be administered in combination with other agents as well, such as, e.g., cells, other proteins or polypeptides or various pharmaceutically-active agents.

In certain aspects, the present invention provides pharmaceutically acceptable compositions which comprise a therapeutically-effective amount of one or more arginase inhibitors of the invention as described herein, formulated together with one or more pharmaceutically acceptable carriers (additives) and/or diluents (e.g., pharmaceutically acceptable cell culture medium).

Particular embodiments of the invention may comprise other formulations, such as those that are well known in the pharmaceutical art, and are described, for example, in Remington: The Science and Practice of Pharmacy, 20th Edition. Baltimore, Md.: Lippincott Williams & Wilkins, 2000.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:
Item 1: A method for diagnosing polycystic ovary syndrome (PCOS) in a subject, the method comprising the steps of:
   (a) Providing one or more biological samples of the subject to be diagnosed,
   (b) Determining in the one or more biological samples one or more of the following biomarkers:
      (i) The level of ornithine, or a metabolic derivative of ornithine, wherein the metabolic derivative of ornithine is indicative for the level of ornithine,
      (ii) The level of arginine, or a metabolic derivative of arginine, wherein the metabolic derivative of arginine is indicative for the level of arginine,
      (iii) The ratio of the levels as determined in (i) and (ii),
      (iv) The level of, or activity of, arginase,
      (v) The level of arginase-bearing microparticles,
      (vi) The presence or absence of a Single Nucleotide Polymorphism (SNP) in an arginase gene;
   (c) Comparing the one or more biomarker determined in (b) with a predetermined references value of said one or more biomarker which is indicative for the absence of PCOS; and/or comparing the one or more biomarker determined in (b) with a reference value of said biomarker determined in a biological sample of a healthy subject;
   wherein if the one or more biomarker as determined in (b) significantly deviates from the reference or control value, the subject is diagnosed with PCOS.
Item 2: The method according to item 1, wherein a level is preferably a concentration.
Item 3: The method according to item 1, which is preferably an *in vitro* or *ex vivo* method.
Item 4: The method according to any one of items 1 to 3, wherein the SNP is a genetic polymorphism in the arginase gene, which increases the expression or activity of arginase in the individual having the SNP compared to an individual not having the SNP.
Item 5: The method according to item 4, wherein the SNP is selected from rs2781666 and rs2781667, preferably of the thymine (t) allele of said SNPs, and more preferably wherein the presence or absence of both SNPs is determined.
Item 6: The method according to any one of items 1 to 5, wherein the one or more biological sample is selected from a tissue sample such as vaginal smear, oocytes, or a liquid sample, such as a blood sample, serum sample or plasma sample.
Item 7: The method according to item 6, wherein for the determining of the biomarkers (i) to (v) a blood sample, or blood-derived sample, and preferably a plasma sample is provided.
Item 8: The method according to item 6 or 7, wherein for the determining of the biomarker (vi) a DNA containing sample is provided, preferably any biological sample comprising genetic material, such as cells, and preferably a sample comprising platelets, preferably wherein the sample is a whole blood sample or buffy coat sample containing leukocytes.
Item 9: The method according to any one of items 1 to 8, wherein for the biomarker in (i) an increase of the level of ornithine, or the metabolic derivative of ornithine, compared to the reference value indicates the presence of PCOS.
Item 10: The method according to any one of items 1 to 9, wherein for the biomarker in (ii) a decrease of the level of arginine, or the metabolic derivative of arginine, compared to the reference value indicates the presence of PCOS.
Item 11: The method according to any one of items 1 to 10, wherein for the biomarker in (iii) an increase of the ratio of [the value determined in (i)] : [the value determined in (ii)] indicates the presence of PCOS.
Item 12: The method according to any one of items 1 to 11, wherein for the biomarker in (iv) an increase of the level of, or activity of, arginase, compared to the reference value indicates the presence of PCOS.
Item 13: The method according to any one of items 1 to 12, wherein for the biomarker in (v) an increase of the level of arginase-bearing microparticles compared to the reference value indicates the presence of PCOS.
Item 14: The method according to any one of items 1 to 13, wherein for the biomarker in (vi) the presence of one of the t alleles of the SNPs, preferably of both, indicates the presence of PCOS.
Item 15: The method according to any one of items 1 to 14, wherein determining the level of the biomarker in (i) to (iii) comprises mass spectrometry, or colorimetric/fluorometric assays.
Item 16: The method according to any one of items 1 to 15, wherein determining the level of the biomarker in (iv) comprises a biochemical assay, such as detection of urea [MAK112®].
Item 17: The method according to any one of items 1 to 16, wherein determining the level of the biomarker in (v) comprises an enzymatic assay, or an immunological assay, such as using an antibody specific for arginase, or detection of arginase mRNA for example using a polymerase chain reaction (PCR) based.
Item 18: The method according to any one of items 1 to 17, wherein determining the level of the biomarker in (vi) comprises nucleic acid hybridization or PCR based assays.
Item 19: The method according to any one of items 1 to 18, wherein the arginase is human arginase 1.
Item 20: A diagnostic kit for the detection of PCOS in a subject, wherein the kit comprises means for the detection of one or more biomarkers selected from, the level of arginine or ornithine, or metabolic derivatives thereof indicative for the level of arginine and/or ornithine, the activity or level of arginase, the level of arginase-bearing microparticles, the presence of an SNP selected from rs2781666 and rs2781667, preferably of both, or combinations thereof, and optionally, instructions for use of the kit.
Item 21: The diagnostic kit according to item 20, for use in a method according to any one of items 1 to 19.
Item 22: An arginase inhibitor for use in the treatment of PCOS.
Item 23: The arginase inhibitor for use according to item 22, wherein the arginase inhibitor is selected from an antibody, or a derivative thereof, a small molecule, a nucleic acid based compound, such as a miRNA, siRNA, gene editing construct(s), or is an amino acid derivative, preferably an arginine or ornithine derivative.
Item 24: The arginase inhibitor for use according to item 22 or 23, wherein the arginase inhibitor when contacted with an arginase enzyme has the ability to reduce the enzymatic activity, or wherein the arginase inhibitor when introduced into a cell reduces the expression of the arginase, for example by reducing activity of the arginase gene, or by interfering with translation (RNA interference).
Item 25: The arginase inhibitor for use according to any one of items 22 to 24, wherein the activity of arginase is catalyzing the reaction: arginine + H2O → ornithine + urea.
Item 26: The arginase inhibitor for use according to any one of items 22 to 25, wherein the arginase inhibitor is 2(S)-amino-6-boronohexanoic acid (ABH), Nω-OH-L-arginine (NOHA), Nω-hydroxy-nor-L-arginine (nor-NOHA), α-difluoromethylornithine (DFMO), L-norvaline, iodoacetyl-L-ornithine, iodoacetyl-L-lysine, L-lysine, and L-citrulline, (2s)-(+)-amino-5-iodoacetamidopentanoic acid, NG-hydroxy-L-arginine, (2S)-(+)-amino-6-iodoacetamidohexanoic acid, and (R)-2-amino-6-borono-2-(2-(piperidin-1 -yl)ethyl)hexanoic acid, the small arginase inhibitor INCB001158 from Calithera or a new small molecule inhibitor of arginase or a combination thereof, and any pharmaceutical acceptable salts or solvates, thereof.
Item 27: The arginase inhibitor for use according to any one of items 22 to 26, wherein the arginase inhibitor in the treatment is administered in a therapeutically effective amount to a subject suffering from, or suspected to suffer from, PCOS.
Item 28: The arginase inhibitor for use according to item 27, wherein the subject is a human, preferably an adult woman.
Item 29: The arginase inhibitor for use according to any one of items 22 to 28, wherein the treatment involves additionally obtaining a biological sample of a subject suspected to suffer from PCOS, diagnosing PCOS in the subject according to the method of any one of items 1 to 16 using said biological sample, and if the subject is diagnosed with PCOS, administering a therapeutically effective amount of the arginase inhibitor to the subject.
Item 30: The arginase inhibitor for use according to any one of items 22 to 29, wherein the treatment involves the steps of monitoring the level or activity of arginase in a biological sample obtained from the subject to be treated, and adjusting the amount of administered arginase inhibitor to reduce the level or activity of arginase in the subject to a therapeutically effective level.
Item 31: The arginase inhibitor for use according to item 30, wherein the therapeutically effective level of arginase level or activity is the level or activity of arginase in a healthy subject.
Item 32: A pharmaceutical composition for use in the treatment of PCOS, comprising an arginase inhibitor and a pharmaceutically acceptable carrier and/or excipient.
Item 33: The pharmaceutical composition according to item 32, wherein the arginase inhibitor is as defined in any of items 22 to 31.
Item 34: The pharmaceutical composition according to item 32, wherein the treatment or use is as defined in any of items 22 to 31.
Item 35: A use of an arginase inhibitor for the manufacturing of a medicament for the treatment of PCOS.
Item 36: The use according to item 35, wherein the arginase inhibitor is as defined in any of the preceding items, and/or wherein the treatment of PCOS is defined as in any one of the preceding items.
Item 37: A method of treatment of PCOS in a subject suffering from or suspected to suffer from PCOS, wherein the method comprises the step of administering to the subject a therapeutically effective amount of an arginase inhibitor.
Item 38: The method according to item 37, wherein the arginase inhibitor is as defined in any of the preceding items, and/or wherein the treatment of PCOS is defined as in any one of the preceding items.
Item 39: The method according to item 37 or 38, wherein the method comprises the steps of obtaining a biological sample of the subject, diagnosing with the biological sample whether the subjects suffers from PCOS according to a method of any one of items 1 to 21, and administering the arginase inhibitor to the subject if PCOS is detected.

### BRIEF DESCRIPTION OF THE FIGURES AND SEQUENCES

The figures show:

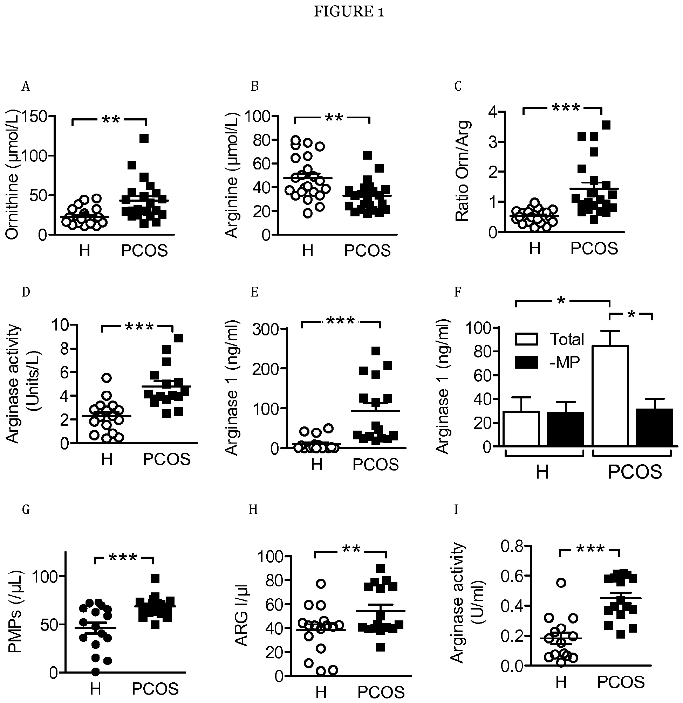

**Figure 1****:** shows the role of platelet-derived microparticles in the alteration of arginine metabolism and arginase activity in samples from PCOS patients. Levels of ornithine (A), arginine (B), and the ratio of ornithine to arginine (C) in plasma from healthy donors or from PCOS patients, n= 22 (Student's t test). (D) Arginase activity in plasma from healthy donors or from PCOS patients, n=16 (Student's t test). (E) Levels of arginase-i in plasma from healthy donors or from PCOS patients, n=16 (Student's t test). (F) Levels of arginase-i in plasma from healthy donors or from PCOS patients in the presence or in the absence of microparticles, n=16 (ANOVA and Newman-Keuls post-test). (G) Levels of platelet-derived microparticles (PMPs) in plasma from healthy donors or from PCOS patients, n=17 (Student's t test). (H) Levels of arginase-i measured by flow cytometry in PMPs from healthy donors and from PCOS patients, n=17. (I) Arginase activity in platelets from healthy donors or from PCOS patients, n=16 (Student's t test). *P<0.05, **P<0.01, ***P<0.001.

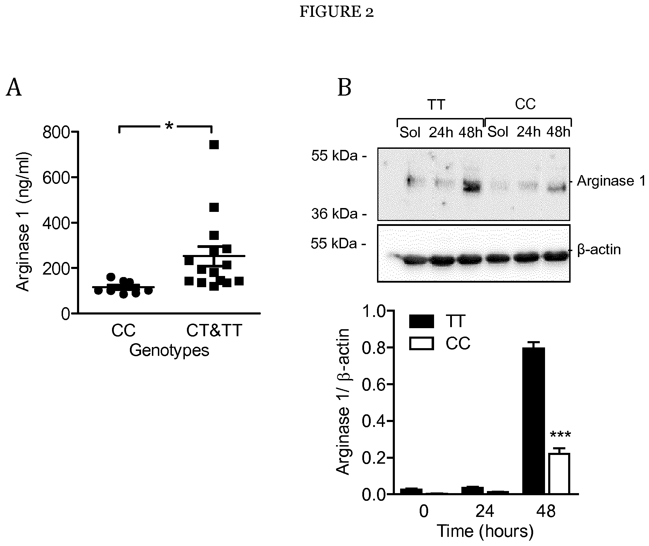

**Figure 2****:** shows the Association between plasma arginase levels and the single nucleotide polymorphism G to T of the rs2781666 and rs2781667 of ARG1 gene. (A) Arginase levels in platelets from CC or TT genotypes donors, n=23 ((Student's t test). (B) Arginase levels after stimulation with interleukin 4 (20 ng/ml, 24 or 48 hours) in macrophages from CC or TT genotypes donors, n=4 (ANOVA and Bonferonni post-test)

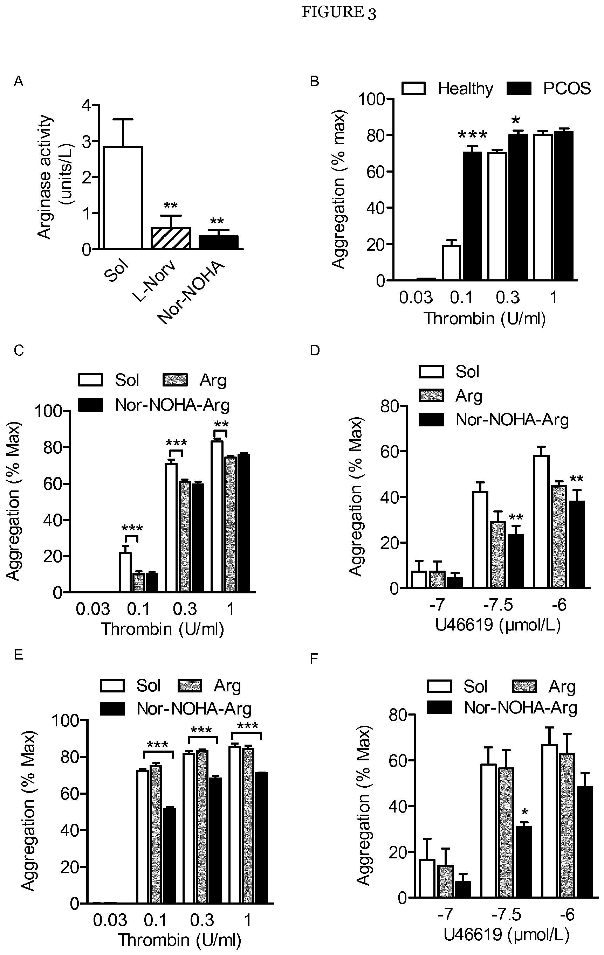

**Figure 3****:** shows the Effect of arginase inhibitors on platelets. (A) Platelets were incubated with the arginase inhibitor L-norvaline (L-norv, 10 mmol/L, 30 minutes) or Nω-hydroxy-nor-Arginine (nor-NOHA, 100 µmol/L, 30 minutes) then lysed and arginase activity was measured. P<0.05. (B) comparison of the aggregation elicited by thrombin on washed platelets from healthy donors and PCOS patients. (C-F) Aggregation elicited by thrombin or by the thromboxane A2 analogue U46619 in washed platelets isolated from healthy donors (C&D) and from PCOS patients (E&F) treated *in vitro* with either arginine (1 mmol/L, 5 minutes) alone or after pre-incubation with the arginase 1 inhibitor Nω-hydroxy-nor-Arginine (Nor-NOHA ,100 µmol/L, 30 minutes).

The sequences show:
**SEQ ID NO: 1 shows the Arginase 1 amino acid sequence:** 
10 20 30 40 50

60 70 80 90 100

110 120 130 140 150

160 170 180 190 200

210 220 230 240 250

260 270 280 290 300

310 320

**SEQ ID NO: 2 shows the Arginase 2 amino acid sequence:**

10 20 30 40 50
MSLRGSLSRL LQTRVHSILK KSVHSVAVIG APFSQGQKRK GVEHGPAAIR
60 70 80 90 100
EAGLMKRLSS LGCHLKDFGD LSFTPVPKDD LYNNLIVNPR SVGLANQELA
110 120 130 140 150
EVVSRAVSDG YSCVTLGGDH SLAIGTISGH ARHCPDLCVV WVDAHADINT
160 170 180 190 200
PLTTSSGNLH GQPVSFLLRE LQDKVPQLPG FSWIKPCISS ASIVYIGLRD
210 220 230 240 250
VDPPEHFILK NYDIQYFSMR DIDRLGIQKV MERTFDLLIG KRQRPIHLSF
260 270 280 290 300
DIDAFDPTLA PATGTPVVGG LTYREGMYIA EEIHNTGLLS ALDLVEVNPQ
310 320 330 340 350
LATSEEEAKT TANLAVDVIA SSFGQTREGG HIVYDQLPTP SSPDESENQA

RVRI

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Arginase activity is increased in PCOS patients

In order to assess the metabolic change associated with PCOS, amino acids levels in plasma from PCOS women were compared with those from healthy controls. Plasma levels of ornithine were significantly higher in samples from PCOS patients than from healthy controls whereas arginine levels were significantly lower (Figure 1A-C). The altered ratio of ornithine to arginine reflects the activity of arginase and plasma arginase activity and levels were indeed significantly increased in samples from PCOS patients (Figure 1 D&E). In order to investigate the possible contribution of microparticles in circulating arginase activity, plasma samples were centrifuged at 20.000 g to remove microparticles. Interestingly, arginase activity and levels were significantly decreased in microparticles-depleted plasma suggesting that arginase was mainly carried by cell-derived microparticles (Figure 1F).

### Example 2: Plasma arginase activity originates from platelets

The inventors next characterized the cellular origin of arginase-bearing microparticles. Given that erythrocytes and the liver are known sources of plasma arginase, the levels of erythrocyte and hepatocyte-derived microparticles in plasma from PCOS patients and healthy donors were compared. The levels of these two types of microparticles were not significantly different between the two groups suggesting that neither erythrocytes nor the liver is likely responsible for the enhanced arginase levels seen in plasma from PCOS.

Given that platelets from PCOS patients are hyperactive and that levels of platelet-derived microparticles have been reported to be enhanced in PCOS, the inventors assessed whether PMPs might contribute to plasma arginase activity. As expected, levels of PMPs were significantly enhanced in PCOS patients (Figure 1G). Moreover, platelets and PMPs from PCOS patients demonstrate significantly enhanced arginase levels and activity (Figure 1H&I) compared to those from healthy donors suggesting that the enhanced arginase activity in PCOS plasma is mainly the consequence of increased circulating arginase-bearing PMPs. Moreover, arginase carried by PMPs is metabolically active converting arginine to ornithine.

### Example 3: The increased arginase expression is associated with the single nucleotide polymorphisms rs2781666 and rs2781667

In order to investigate whether the change in arginase levels is associated with a genetic variant, SNP analysis of the arginase was assessed. Among 18 SNPs tested, the frequencies for the allele T of the rs2781666 and rs2781667 were significantly higher in PCOS patients compared to the healthy donors (76,82% vs 45,54%). Moreover, the allele T was significantly associated with enhanced arginase expression (Figure 2A).

In order to further investigate the link between the genotype T and arginase expression, macrophages from C and T genotypes donors were stimulated with IL-4 to induce arginase expression and Western blot was performed to quantify arginase expression. The arginase expression was significantly higher in macrophages from T genotypes (Figure 2B).

### Example 4: Arginase inhibitor is a potential new therapeutical approach to treat PCOS

Given that platelets are the main source of plasma arginase in PCOS patients, the effect of arginase inhibitor on platelet arginase activity and function was assessed. Treatment of platelets with the arginase inhibitors L-norvaline or Nω-hydroxy-nor-Arginine (NorNOHA) significantly inhibited arginase activity (Figure 3A).

Platelets from PCOS patients demonstrated increased aggregation in response to agonists such as thrombin (Figure 3B). Addition of arginine inhibited thrombin or thromboxane A2 analogue-induced aggregation of platelets from healthy donors but did not affect the aggregation of platelets from PCOS patients. However, pre-treatment of platelets with the arginase inhibitors L-norvaline or NorNOHA significantly revealed the inhibitory effects of arginine on the aggregation of platelets from PCOS patients whereas they did not have additional effect on arginine-mediated platelet inhibition in healthy donors (Figure 3C-F).

### Conclusions

The results of this study indicate that hyperactive platelets release high levels of arginase-bearing microparticles which largely contribute to the increase in plasma arginase activity and the alteration of arginine metabolism in women with PCOS.

l-Arginine is a semi-essential amino acid synthesized from citrulline via the intestinal-renal axis. Plasma arginine is metabolized through different pathways including arginine decarboxylase, arginase and nitric-oxide (NO) synthase to produce urea, ornithine and NO, respectively [9]. Arginase and NO synthases compete for the same substrate, which means that an increase in plasma arginase activity would deplete the NO synthases of their substrate to promote endothelial dysfunction at the same time as increasing the production of ornithine. Moreover, given that the decrease in arginine and NO bioavailability is known to largely contribute to insulin resistance, the associated compensatory hyperinsulinemia may explain the increase in androgen levels in PCOS patients. Indeed, insulin can contribute to androgenemia in different ways including a direct effect on the thecal and granulosa cells or indirectly by decreasing the synthesis of sexual hormon-binding-globulin from the liver resulting in the increase of free testosterone. The increase in arginase activity can account for the lack of cardiovascular benefit of arginine supplementation in conditions such as acute myocardial infarction [10]. Arginase is an intracellular enzyme that has been believed to appear in the plasma only after cell damage. However, there is evidence that arginase is also carried by cell-derived vesicles which are released during cell activation. It is well known that more than 60% of circulating microparticles derive from platelets and their levels are known to be enhanced in different pathological conditions including PCOS. The present study reported that not only PMP levels were significantly increased in plasma from PCOS patients but they also carry high levels of active arginase responsible of the alteration of arginine metabolism. Furthermore, the enhanced arginase levels were very likely the consequence of an enhanced transcription of the arginase gene bearing the allele T of the SNP 2781666 and 2781667. Finally, the improvement of platelet function by the arginase inhibitors suggest that these compounds are promising new therapies for the treatment of PCOS patients. By improving arginine bioavailability, arginase inhibitors will not only enhance insulin sensitivity but also improve endothelial and platelet function and prevent the development of cardiovascular diseases. Further studies with a higher number of participants are planned to confirm the present finding.

### Materials and Methods:

### Healthy donors and PCOS patients

A total of thirty treatment-naive PCOS patients (mean age 29.34±1.25 years; BMI 26.08±1.38 Kg/m2, fasting plasma glucose 88.32±1.62, HOMA-IR 2.28±0.35) attending the clinic for fertility problems were included in the present study. Thirty age-matched female subjects (mean age 30.39±1.27 years; BMI 20.86±0.49 Kg/m2) without PCOS or insulin resistance served as the control group. None of the participants took any food supplements or medication known to interfere with platelet aggregation for at least 10 days before fasting blood sampling. The study protocol was approved by the ethics committee of the Goethe University Hospital (No. E 61/09 Geschäfts Nr 86/09) and the Landesärztekammer Hessen. All of the participants gave written informed consent.

### Platelet isolation and plasma sampling

Human platelets were obtained by centrifugation (900g, 7 minutes) of platelet-rich plasma, as described [8]. The resulting pellet was washed in Ca²⁺-free HEPES buffer (mmol/L: NaCl, 136; KCl, 2.6; MgCl2, 0.93; NaH2PO4, 3.26; glucose, 5.5; HEPES, 3.7; pH 7.4 at 37°C) and used for further experiments. The resulting platelet-poor plasma was collected and frozen at -80°C for further evaluation.

### Quantification of free amino acids in plasma

Plasma samples (100 µL) were used for amino acid analysis. Sample preparation was performed using the EZ:faast LC MS free amino acid analysis kit (Phenomenex, Aschaffenburg, Germany) according to the manufacturer's instructions, with minor modifications. Internal standards (10 µL) were applied to all samples and to the standard curve. Analysis of metabolites was performed by LC-MS/MS using the EZ:faast AAA-MS HPLC column on an Agilent 1290 Infinity LC system (Agilent, Waldbronn, Germany) coupled to a QTrap 5500 mass spectrometer (Sciex, Darmstadt, Germany). Electro spray ionization in positive mode was employed. The intensity of the measured metabolite was normalized to internal standards. Analyst 1.6.2 and MultiQuant 3.0 (Sciex, Darmstadt, Germany), were used for data acquisition and analysis, respectively.

### Measurement of arginase activity

Arginase activity was determined in plasma or in platelets using commercially available kits (MAK112, Sigma, Steinheim, Germany), according to manufacturer's instructions.

Platelet aggregation. Aggregation of washed human platelets (4x108platelets/mL) was measured using an 8-channel aggregometer (PAP8, Mölab, Germany).

### Cell culture

Monocytic cells were isolated from human whole blood and kept in culture for 7 days (RMPI+MEM) to allow differentiation into macrophages. Thereafter, macrophages were polarized to M2 type by stimulation with interleukin 4 (20ng/ml) for up to 48 hours to induce the expression of arginase 1.

### Immunoblotting

Cells were lysed in triton X100 and solubilized in SDS sample buffer, heated at 95°C and proteins were separated by SDS-PAGE and subjected to Western blotting and visualized by enhanced chemiluminescence using a commercially available kit (Amersham, Freiburg, Germany), as described [8].

### DNA isolation and genotyping

DNA was extracted from peripheral blood mononuclear cells isolated from healthy donors or PCOS patients and single nucleotide polymorphism (SNP) of the arginase gene was detected by multiplexed massarray system (Agena Biosciences, Germany)

### Statistical analysis

Data are expressed as mean ± SEM and statistical evaluation was performed using Student's t test for unpaired data using Prism software (GraphPad). Values of P<0.05 were considered statistically significant.

### REFERENCES

The references are:
[1] Bozdag G, Mumusoglu S, Zengin D et al. The prevalence and phenotypic features of polycystic ovary syndrome: a systematic review and meta-analysis. Hum.Reprod. 2016; 31: 2841- 2855.
[2] Skiba MA, Islam RM, Bell RJ, Davis SR. Understanding variation in prevalence estimates of polycystic ovary syndrome: a systematic review and meta-analysis. Hum.Reprod.Update. 2018; 24: 694-709
[3] Escobar-Morreale HF. Polycystic ovary syndrome: definition, aetiology, diagnosis and treatment. Nat Rev Endocrinol. 2018; 14:270-284.
[4] Sathyapalan T, Atkin SL. Recent advances in cardiovascular aspects of polycystic ovary syndrome. Eur J Endocrinol. 2012; 166: 575- 583.
[5] Roque M, Tostes AC, Valle M, Sampaio M, Geber S. Letrozole versus clomiphene citrate in polycystic ovary syndrome: systematic review and meta-analysis. Gynecol Endocrinol 2015; 31: 917- 921.
[6] Yu Y, Fang L, Zhang R, He J, Xiong Y, Guo X, Du Q, Huang Y, Sun Y. Comparative effectiveness of 9 ovulation-induction therapies in patients with clomiphene citrate-resistant polycystic ovary syndrome: a network meta-analysis. Sci Rep 2017; 7: 3812
[7] Diaz R. Reproductive endocrinology: Benefits of metformin in PCOS. Nat Rev Endocrinol 2011; 7: 437
[8] Randriamboavonjy V, Schrader J, Busse R et al. Insulin induces the release of vasodilator compounds from platelets by a nitric oxide-G kinase-VAMP-3-dependent pathway. J Exp Med. 2004; 199: 347- 356.
[9] Wu G, Bazer FW, Davis TA et al. Arginine metabolism and nutrition in growth, health and disease. Amino Acids 2009; 37: 153- 168.
[10] Schulman SP, Becker LC, Kass DA et al. L-arginine therapy in acute myocardial infarction: the Vascular Interaction With Age in Myocardial Infarction (VINTAGE MI) randomized clinical trial. JAMA 2006; 295: 58- 64.

## Claims

1. **A method for diagnosing polycystic ovary syndrome (PCOS) in a subject,** the method comprising the steps of:
**(a)** Providing one or more biological samples of the subject to be diagnosed,
**(b)** Determining in the one or more biological samples one or more of the following biomarkers:
(i) The level of ornithine, or a metabolic derivative of ornithine, wherein the metabolic derivative of ornithine is indicative for the level of ornithine,
(ii) The level of arginine, or a metabolic derivative of arginine, wherein the metabolic derivative of arginine is indicative for the level of arginine,
(iii) The ratio of the levels as determined in (i) and (ii),
(iv) The level of, or activity of, arginase,
(v) The level of arginase-bearing microparticles,
(vi) The presence or absence of a Single Nucleotide Polymorphism (SNP) in an arginase gene;
**(c)** Comparing the one or more biomarker determined in (b) with a predetermined references value of said one or more biomarker which is indicative for the absence of PCOS; and/or comparing the one or more biomarker determined in (b) with a reference value of said biomarker determined in a biological sample of a healthy subject;
wherein if the one or more biomarker as determined in (b) significantly deviates from the reference or control value, the subject is diagnosed with PCOS.

2. The method according to claim 1, wherein the SNP is a genetic polymorphism in the arginase gene, which increases the expression or activity of arginase in the individual having the SNP compared to an individual not having the SNP.

3. The method according to claim 2, wherein the SNP is selected from rs2781666 and rs2781667, preferably of the thymine (t) allele of said SNPs, and more preferably wherein the presence or absence of both SNPs is determined.

4. The method according to any one of claims 1 to 3, wherein the one or more biological sample is selected from a tissue sample such as vaginal smear, oocytes, or a liquid sample, such as a blood sample, whole blood sample, serum sample or plasma sample.

5. The method according to any one of claims 1 to 4, wherein the arginase is human arginase 1.

6. **A diagnostic kit for the detection of PCOS in a subject,** wherein the kit comprises means for the detection of one or more biomarkers selected from, the level of arginine or ornithine, or metabolic derivatives thereof indicative for the level of arginine and/or ornithine, the activity or level of arginase, the level of arginase-bearing microparticles, the presence of an SNP selected from rs2781666 and rs2781667, preferably of both, or combinations thereof, and optionally, instructions for use of the kit.

7. **An arginase inhibitor for use in the treatment of PCOS.**

8. The arginase inhibitor for use according to claim 7, wherein the arginase inhibitor is selected from an antibody, or a derivative thereof, a small molecule, a nucleic acid based compound, such as a miRNA, siRNA, gene editing construct(s), or is an amino acid derivative, preferably an arginine or ornithine derivative.

9. The arginase inhibitor for use according to claim 7 or 8, wherein the arginase inhibitor when contacted with an arginase enzyme has the ability to reduce the enzymatic activity, or wherein the arginase inhibitor when introduced into a cell reduces the expression of the arginase, for example by reducing activity of the arginase gene, or by interfering with translation (RNA interference).

10. The arginase inhibitor for use according to any one of claims 7 to 9, wherein the arginase inhibitor is 2(S)-amino-6-boronohexanoic acid (ABH), Nω-OH-L-arginine (NOHA), Nω-hydroxy-nor-L-arginine (nor-NOHA), α-difluoromethylornithine (DFMO), L-norvaline, iodoacetyl-L-ornithine, iodoacetyl-L-lysine, L-lysine, and L-citrulline, (2s)-(+)-amino-5-iodoacetamidopentanoic acid, NG-hydroxy-L-arginine, (2S)-(+)-amino-6-iodoacetamidohexanoic acid, and (R)-2-amino-6-borono-2-(2-(piperidin-1-yl)ethyl)hexanoic acid, the small arginase inhibitor INCB001158 from Calithera or a new small molecule inhibitor of arginase or a combination thereof, and any pharmaceutical acceptable salts or solvates, thereof.

11. The arginase inhibitor for use according to any one of claims 7 to 10, wherein the arginase inhibitor in the treatment is administered in a therapeutically effective amount to a subject suffering from, or suspected to suffer from, PCOS.

12. The arginase inhibitor for use according to any one of claims 7 to 11, wherein the treatment involves additionally obtaining a biological sample of a subject suspected to suffer from PCOS, diagnosing PCOS in the subject according to the method of any one of claims 1 to 5 using said biological sample, and if the subject is diagnosed with PCOS, administering a therapeutically effective amount of the arginase inhibitor to the subject.

13. The arginase inhibitor for use according to any one of claims 7 to 12, wherein the treatment involves the steps of monitoring the level or activity of arginase in a biological sample obtained from the subject to be treated, and adjusting the amount of administered arginase inhibitor to reduce the level or activity of arginase in the subject to a therapeutically effective level.

14. **A pharmaceutical composition for use in the treatment of PCOS,** comprising an arginase inhibitor and a pharmaceutically acceptable carrier and/or excipient.

15. The pharmaceutical composition according to claim 14, wherein the arginase inhibitor is as defined in any of claims 7 to 13, and/or wherein the treatment or use is as defined in any of claims 7 to 13.


SEQUENCE LISTING

<110>  Johann Wolfgang Goethe-Universität Frankfurt am Main

<120>  ARGINASE AS BIOMARKER AND DRUG TARGET FOR PCOS

<130>  U30907EP

<160>  2

<170>  PatentIn version 3.5

<210>  1
<211>  322
<212>  PRT
<213>  Homo sapiens

<400>  1

<210> 2
<211> 354
<212> PRT
<213> Homo sapiens

<400> 2

Met Ser Leu Arg Gly Ser Leu Ser Arg Leu Leu Gln Thr Arg Val His
1               5                   10                  15

Ser Ile Leu Lys Lys Ser Val His Ser Val Ala Val Ile Gly Ala Pro
            20                  25                  30

Phe Ser Gln Gly Gln Lys Arg Lys Gly Val Glu His Gly Pro Ala Ala
        35                  40                  45

Ile Arg Glu Ala Gly Leu Met Lys Arg Leu Ser Ser Leu Gly Cys His
    50                  55                  60

Leu Lys Asp Phe Gly Asp Leu Ser Phe Thr Pro Val Pro Lys Asp Asp
65                  70                  75                  80

Leu Tyr Asn Asn Leu Ile Val Asn Pro Arg Ser Val Gly Leu Ala Asn
                85                  90                  95

Gln Glu Leu Ala Glu Val Val Ser Arg Ala Val Ser Asp Gly Tyr Ser
            100                 105                 110

Cys Val Thr Leu Gly Gly Asp His Ser Leu Ala Ile Gly Thr Ile Ser
        115                 120                 125

Gly His Ala Arg His Cys Pro Asp Leu Cys Val Val Trp Val Asp Ala
    130                 135                 140

His Ala Asp Ile Asn Thr Pro Leu Thr Thr Ser Ser Gly Asn Leu His
145                 150                 155                 160

Gly Gln Pro Val Ser Phe Leu Leu Arg Glu Leu Gln Asp Lys Val Pro
                165                 170                 175

Gln Leu Pro Gly Phe Ser Trp Ile Lys Pro Cys Ile Ser Ser Ala Ser
            180                 185                 190

Ile Val Tyr Ile Gly Leu Arg Asp Val Asp Pro Pro Glu His Phe Ile
        195                 200                 205

Leu Lys Asn Tyr Asp Ile Gln Tyr Phe Ser Met Arg Asp Ile Asp Arg
    210                 215                 220

Leu Gly Ile Gln Lys Val Met Glu Arg Thr Phe Asp Leu Leu Ile Gly
225                 230                 235                 240

Lys Arg Gln Arg Pro Ile His Leu Ser Phe Asp Ile Asp Ala Phe Asp
                245                 250                 255

Pro Thr Leu Ala Pro Ala Thr Gly Thr Pro Val Val Gly Gly Leu Thr
            260                 265                 270

Tyr Arg Glu Gly Met Tyr Ile Ala Glu Glu Ile His Asn Thr Gly Leu
        275                 280                 285

Leu Ser Ala Leu Asp Leu Val Glu Val Asn Pro Gln Leu Ala Thr Ser
    290                 295                 300

Glu Glu Glu Ala Lys Thr Thr Ala Asn Leu Ala Val Asp Val Ile Ala
305                 310                 315                 320

Ser Ser Phe Gly Gln Thr Arg Glu Gly Gly His Ile Val Tyr Asp Gln
                325                 330                 335

Leu Pro Thr Pro Ser Ser Pro Asp Glu Ser Glu Asn Gln Ala Arg Val
            340                 345                 350

Arg Ile